# EUROPEAN PATENT APPLICATION

(11) **EP 4 316 397 A1**
(43) Date of publication of application: **07.02.2024**
(21) Application number: 23185677.4
(22) Date of filing: 14.07.2023
(51) Int. Cl.: A61B 18/14, A61B 18/04, A61B 18/12, A61B 18/00

(54) **EROSION FUSE TO REDUCE RISK OF RF TIP DETACHMENT**

(30) Priority: 02.08.2022 US 202263394470 P
(71) Applicant: Gyrus Medical Limited, Cardiff CF3 0LT (GB)
(72) Inventor: DICKSON, James Alan, Cardiff, CF3 0LT (GB)
(74) Representative: Wallin, Nicholas James

(57) **Abstract**

The present disclosure relates to a RF hand instrument wherein the RF ablation function ceases to operate prior to any RF tip detachment occurs, in order for the hand instrument to fail gracefully and thus be considered fail-safe. This is achieved by ensuring there is a mechanical retention mechanism which is separate to the RF power delivery means such that the power is delivered by a separate electro/mechanical connection. Further, the electrical connection that is used to deliver RF power to the active tip repeatably and reliably erodes at a faster rate than the active tip and the active tip retention mechanism such that the electrical connection geometry erodes to the point where RF power is no longer delivered to the RF tip, and the user will be made aware of this loss in RF function. Thus, the geometry of the used/eroded RF tip, with no remaining electrical connection is still mechanically strong enough to be removed from the patient or cannula without RF tip detachment from the electrode assembly.

## Description

### Technical Field

The present disclosure generally relates to radio frequency (RF) electrosurgical hand instruments for use in completing ablation functions or for use in surgical procedures such as arthroscopy or for treating tissue, tumors, lesions or tissue masses. Further, the present disclosure relates to the use of RF energy for material ablation with increased patient safety and fail-safe design.

The present disclosure further relates to RF ablation surgical hand instruments to perform the ablation function which incorporates a fail-safe operation to prevent RF tip detachment during use.

### Background and Related Art

A number of legacy wet-field RF electrosurgical hand instruments (such as GB2598332A or alike) for arthroscopy have an RF tip which is both mechanically and electrically connected to the rest of the shaft assembly via various means. Typically, this is through a mechanical interlock which doubles as an electrical connection, or via a mechanical interlock/adhesive with a secondary connection to an active RF wire, to transmit RF energy.

In the legacy configurations, the steel components of the RF tip and active inner tube start to erode due to interaction with RF plasma and saline during ablation in normal use. If either device is forced past its 'normal use' and into 'mis-use' by ablating for a very long time, the erosion will eventually be high enough to cause the RF tip to fragment into separate pieces. The exact nature of the fragmentation and failure depends on the clinical loads, saline flow rate, RF tip power and other factors.

Importantly, both electrical and mechanical failure between the active tube and the RF tip typically occur at the same instant. Therefore, the design does not fail gracefully, but fails both in RF performance terms, and mechanically with detached tip fragments. Leaving tip fragments within the patient at the surgical site can have deleterious effects on the patient's recovery.

### Summary of the Invention

The present disclosure relates to a RF electrosurgical hand instrument wherein the RF ablation function ceases to operate prior to the occurrence of any mechanical RF tip detachment, in order to allow the hand instrument to fail gracefully and thus be considered to fail-safe.

The present disclosure incorporates the following design characteristics to achieve the fail-safe RF hand instrument. A mechanical retention mechanism (keeping RF active tip inside the distal end) which is mechanically separate to the RF power delivery means. The RF power delivery means delivers the RF power to the active RF tip by a separate electro/mechanical connection (e.g - wire). The electrical connection used to deliver RF power to the active tip repeatably and reliably erodes at a faster rate than the active tip and the active tip retention mechanism, as will be discussed in further detail throughout the description. The electrical connection geometry erodes to the point where RF power is no longer delivered to the RF tip i.e., to the point wherein the electrical circuit is broken and can therefore no longer deliver RF power to the active tip. At this point, the user will be made aware of this loss in RF function. The geometry of the used/eroded RF tip, with no remaining electrical connection is still mechanically strong enough to be removed from the patient or cannula without RF tip detachment from the electrode assembly.

The present disclosure has the advantages of preventing the device from failing both in RF performance and mechanically with detached tip fragments. Therefore, the disclosure provides a large advantage over the prior art by reducing the chance of detached RF tip fragments being able to enter or injure the patient. This will reduce the risk of further injury or complications to the patient during ablation procedures or alike.

The present invention provides a method and system according to the appended claims.

A first aspect of the teachings provides an electrosurgical end effector, comprising any or or all of the following: a tissue treatment electrode mounted in an insulative instrument tip, the end effector further comprising an electrical connection to supply RF energy to the tip of the tissue treatment electrode, the arrangement being such that the electrical connection is arranged to fail in use prior to any failure of the tissue treatment electrode.

The electrical connection may erode in use faster than the tissue treatment electrode.

The tissue treatment electrode may be separate to the electrical connection.

The electrical connection may be made from an electrically conductive material which erodes faster than the RF tip in the same plasma conditions.

The electrical connection may have a greater area exposed to RF plasma by virtue of the insulative instrument tip geometry.

In an arrangement, there may be an enlarged open window over the electrical connection is present in the insulative instrument tip to promote increased erosion levels in this area compared to the tissue treatment electrode.

The electrical connection may have a thin geometry.

The end effector may further comprise a saline suction passageway which draws hot saline over the electrical connection to increase erosion levels in this area compared to the tissue treatment electrode.

The tip of the tissue treatment electrode may still be mechanically strong after the electrical connection has fully eroded, such that the tissue treatment electrode may be removed from the patient without the tip of the tissue treatment electrode becoming detached.

The components of the tissue treatment electrode may be welded together in a configuration that prevents the removal of the tissue treatment electrode from the insulative instrument tip.

A user may be alerted when the electrical connection has been fully eroded, and RF function has been lost.

The electrical connection may erode to failure in use beyond a time considered normal use.

A second aspect of the teachings provides an RF hand instrument tip configuration, comprising any or all of the following: a RF tip and a retention means mounted in a ceramic insulator, wherein, the RF tip and the retention means further comprise an electrical connection to supply RF energy to the RF tip, the arrangement being such that the electrical connection reliably erodes at a faster rate than the RF tip or retention means.

The retention means may comprise an RF tip retainer and an RF tip retention mechanism.

The electrical connection may be made to reliably erode faster than the RF tip and retention means by: making the electrical connection from an electrically conductive material which erodes faster than the RF tip in the same plasma conditions; and/or designing the ceramic insulator geometry such that an enlarged open window over the electrical connection is present to promote increased erosion levels in this area; and/ordesigning the electrical connection to have a thin or narrow geometry; and/or designing a saline suction passageway which draws hot saline over the electrical connection to increase erosion levels in this area.

A third aspect of the teachings provides a method for processing an instrument for surgery, the method comprising any or all of the following: obtaining the electrosurgical end effector of any of the preceding claims; reprocessing the electrosurgical end effector; sterilizing the electrosurgical end effector; and storing the electrosurgical end effector in a sterile container.

### Brief Description of the Drawings

Examples of the present disclosure are now described with reference to the accompanying drawings, wherein like reference numerals refer to like parts, in which:
Figure 1 shows a cross-sectional diagram from a perspective view of a legacy device.
Figure 2a shows an image of an RF tip configuration before an erosion test.
Figure 2b shows an image of a RF tip configuration mid-way through an erosion test at the normal use limit.
Figure 2c shows an image of a RF tip configuration after an erosion test wherein the device has been pushed beyond the normal use limit.
Figure 3 shows a cross-sectional diagram from a perspective view of the arrangement of the present invention.
Figure 4a shows a cross-sectional diagram from a perspective view of the arrangement of the present invention according to a further embodiment.
Figure 4b shows a cross-sectional diagram from a perspective view of the arrangement of the present invention.
Figure 4c shows a cross-sectional diagram from a perspective view of the arrangement of the present invention.
Figure 5 is a flowchart indicating a reprocessing method for the treatment instrument.

### Detailed Description of the Preferred Embodiments

The present disclosure relates to a RF electrosurgical hand instrument wherein the RF ablation function ceases to operate prior to the occurrence of any mechanical RF tip detachment, in order for the hand instrument to fail gracefully and thus be considered fail-safe. This is achieved by ensuring there is a mechanical retention mechanism which is separate to the RF power delivery means such that the power is delivered by a separate electrical connection than the mechanical retention mechanism. Further, the electrical connection that is used to deliver RF power to the active tip repeatably and reliably erodes at a faster rate than the active tip and the active tip retention mechanism such that the electrical connection geometry erodes to the point where RF power is no longer delivered to the RF tip, and the user will be made aware of this loss in RF function. Thus, the geometry of the used/eroded RF tip, with no remaining electrical connection is still mechanically strong enough to be removed from the patient or cannula without RF tip detachment from the electrode assembly.

Further, the electrical connection erodes at a faster rate than the steel tip retaining features that mechanically retain the treatment tip within the instrument. Therefore, the treatment tip remains attached to the rest of the instrument even after the copper conductor providing the electrical connection to the tip erodes through. The relative rate of preferential erosion of the active wire (or other electrical conduction member) compared to the RF active tip itself can be tuned to achieve the desired fail-safe outcome in a plurality of ways.

In a first embodiment, the rate of preferential erosion of the electrical connection may be achieved by deliberately exposing a greater surface of the electrical connection to the RF plasma present during a surgical procedure. This is achieved by virtue of the ceramic insulator geometry, wherein a window or hole is left in the ceramic insulator such that the electrical connection is uncovered and open. This deliberate exclusion of insulation near the electrical connection will result in an increased rate of erosion in comparison to other elements of the end effector.

In a second embodiment, the rate of preferential erosion of the electrical connection may be achieved by deliberately choosing an electrical conductor material which erodes faster than the RF active tip in the same plasma conditions. For example, a copper wire and steel or tungsten tip could be used. In this arrangement, the copper will erode at a faster rate than the tungsten, due to their respective material properties. As such, the copper wire would fully erode before the tungsten tip, therefore, breaking the electrical circuit and stopping RF function to the active RF tip before the tungsten tip erodes to a level that could result in detachment.

In a third embodiment, the rate of preferential erosion of the electrical connection may be achieved by deliberately designing the geometry of the electrical connection to be thin or narrow in places. This would ensure the electrical connection acts like an 'erosion fuse' when the RF active tip is pushed beyond normal use, as the electrical connection would repeatably and reliably erode fastest in the area of thinnest or narrowest geometry. In this arrangement, the RF active tip and the electrical connection could therefore be made of the same material if preferred as the geometry of the electrical connection will be the key component in repeatable and reliable erosion rather than the respective material properties.

In a fourth embodiment, the rate of preferential erosion of the electrical connection may be achieved by deliberately designing the geometry between the active and return electrodes. This would be designed to focus current density at the location of the electrical connection to the RF active tip. For example, the arrangement may have a shorter RF tracking distance over the ceramic insulator nearest the electrical connection to obtain preferential erosion in this area.

In a fifth embodiment, the rate of preferential erosion of the electrical connection may be achieved by deliberately designing the geometry of the end effector such that a saline suction passageway (peripheral or main RF suction is present. The saline suction passageway would ensure that hot saline is intentionally, and in a controlled manner, drawn over the electrical connection area. In this arrangement, the intentional drawing of hot saline over the electrical connection area will cause the electrical connection to erode at an increased rate in comparison to the other areas of the end effector.

It is worth noting that despite the different methods of ensuring faster, more reliable and repeatable electrical connection erosion being explained in different embodiments; the embodiments can be combined in a multitude of possible combinations. An example combination could include the second and third embodiment. Therefore, this combination would include choosing an electrical connection conductor material which erodes faster than the RF active tip in the same plasma conditions i.e., a copper wire and steel or tungsten tip could be used; and designing the geometry of the electrical connection to be thin or narrow in places, to ensure the electrical connection acts like an 'erosion fuse'. This example combination could be further combined with the first embodiment, wherein a greater surface area of the electrical connection would be exposed to the RF plasma during operation.

In a further arrangement, but similar to the first embodiment, a more robust mechanical tip interlock could be created with a double-undercut feature. While a more exposed active wire would promote faster, more repeatable and more reliable erosion (to be tuned according to defined electrode lifespan). This configuration provides the further advantages of increasing the mechanical retention and interlocking geometry, while deliberately exposing more of the active wire to plasma, and therefore erosion. This should help to ensure that the electrical connection always fails prior to the mechanical interlock.

Further, the present invention further provides an electrosurgical end effector, wherein the end effector contains a tissue treatment electrode which is mounted within an insulative instrument tip. The end effector further comprises an electrical connection to supply the tip of the tissue treatment electrode with RF energy. In this arrangement, the electrical connection is designed to fail in use prior to any failure of the tissue treatment electrode. In this respect, the tissue treatment electrode corresponds to the RF tip, RF tip retainer and RF tip retention mechanism. The insulative instrument tip corresponds to the ceramic insulator.

The tip/ceramic geometry, waveform, power, voltage limits, and suction characteristics are tuned and/or optimised to ensure that:
- The active wire preferentially erodes first in all normal usage conditions; and
- The active wire erodes at a time beyond what is considered 'normal use' of the electrode, so that the planned failure does not lead to an increase in complaints.

Further details of an embodiment of the present disclosure will now be described with respect to the figures.

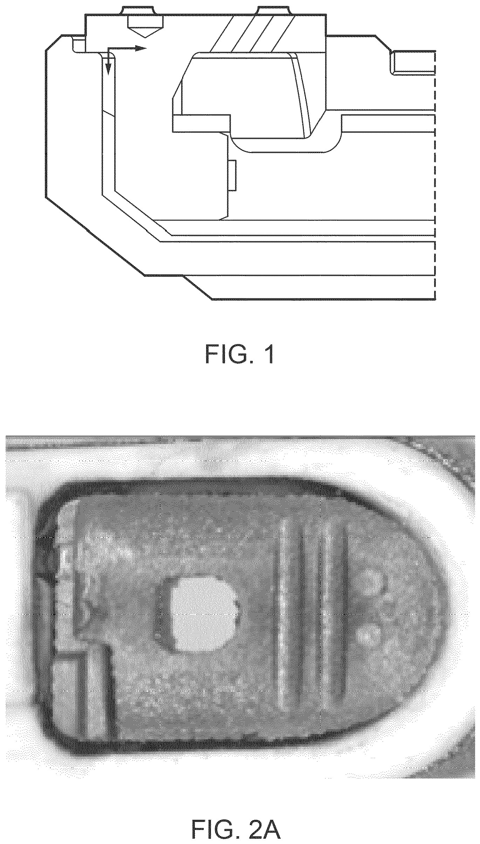

Figure 1 shows a cross-sectional diagram of a legacy device configuration such as in GB2598332A. The diagram shows a combined mechanical and electrical connection between the active tip and an active suction tube. In this configuration, the steel components of the RF tip and active inner tube start to erode due to interaction with RF plasma and saline during ablation in normal use. If either device is forced past its 'normal use' and into 'mis-use' by ablating for a very long time, the erosion will eventually be high enough to cause the RF tip to fragment into separate pieces. The exact nature of the fragmentation and failure depends on the clinical loads, saline flow rate, RF tip power and other factors. The point to note is that both electrical and mechanical failure between the active tube and the RF tip occur at the same instant. Therefore, the design does not fail gracefully, but fails both in RF performance terms, and mechanically with detached tip fragments.

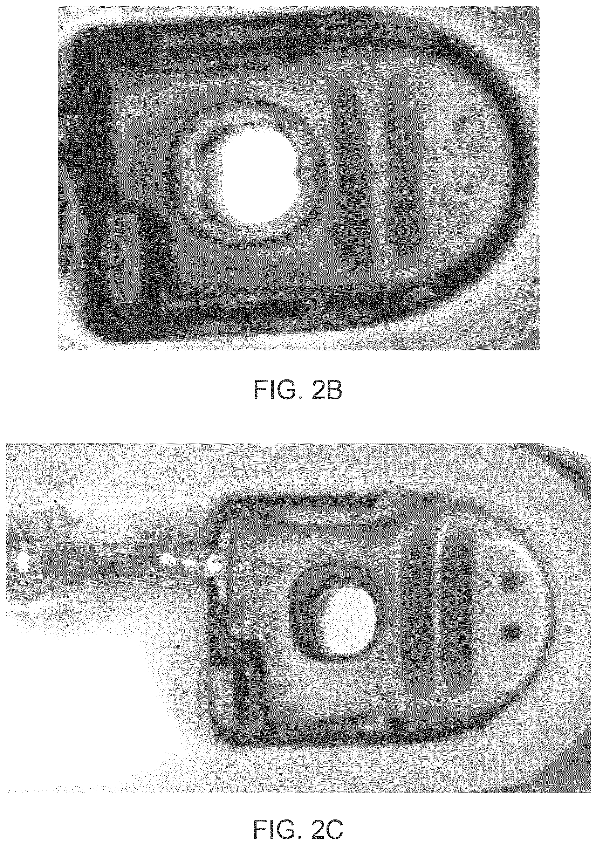

Figure 2a shows an image of an RF tip configuration prototype of the present disclosure before an erosion test. This shows the RF tip configuration as it should appear during low usage or periods of normal use. Figure 2b shows the same RF tip configuration prototype during the erosion test. In which the prototype has been used up to the normal use limit. Beyond this point, in legacy configurations non-graceful failure would occur in which the RF tip could fragment and cause damage to the patient. In the present disclosure this should not occur, and the device should fail-safe meaning the device would cease to complete ablation function by erosion of the electrical connection before the erosion of the active RF tip or the active RF tip retention mechanism. Figure 2c shows the same RF tip configuration prototype after the erosion test. At this point, the prototype has been pushed beyond normal use into misuse or abnormal usage. However, it can be seen that the electrical connection has failed as the copper wire, shown on the left-hand side, has eroded away to break the electrical circuit. The RF active tip remains attached to the ceramic insulator via the mechanical interlocks with the ceramic. Therefore, this would allow for the removal of the instrument after such a misuse erosion scenario with no additional patient risk due to the detachment of tip fragments. A second device could then be used to complete the procedure safely as required.

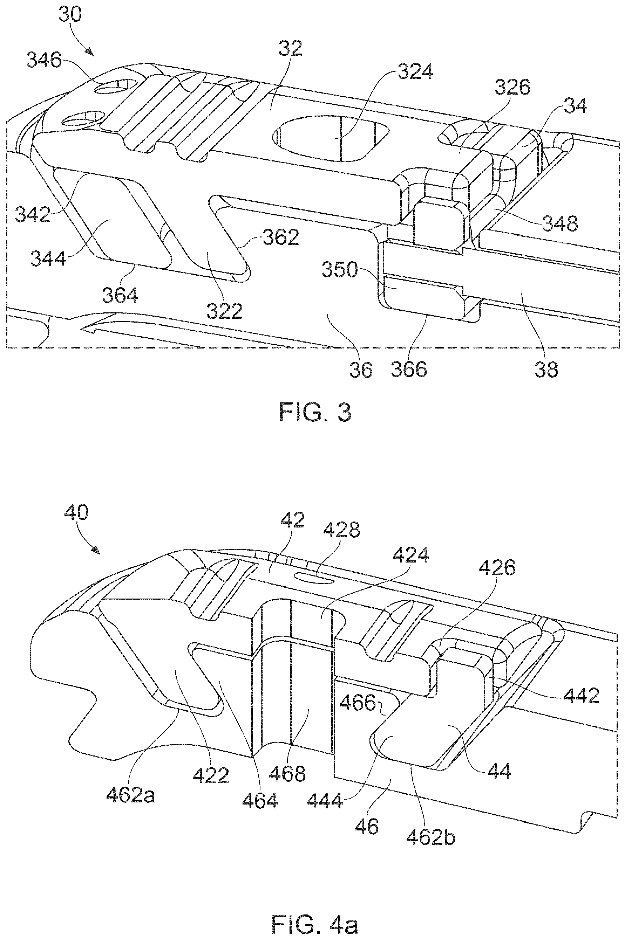

Figure 3 shows a cross-sectional diagram of the RF hand instrument of the present disclosure, which depicts the RF tip retention mechanism, the RF tip retainer and the electrical connection which in this case is a copper wire. Further, the RF tip retention mechanism and RF tip retainer are welded together. In this arrangement, the RF tip retention mechanism and RF tip retainer are inserted into the ceramic base via an undercut connection at front of the instrument. The RF tip retainer is further inserted into the ceramic base at the rear of the instrument, where it is connected to the electrical connection.

Further, the RF hand instrument 30 or electrosurgical end effector 30 shown in Figure 3, is composed of the RF tip retention mechanism 32, the RF tip retainer 34, the ceramic insulator 36 and the electrical connection 38. The RF tip retention mechanism 32 consists of a front undercut protrusion 322 arrangement, a hole or passageway 324 and a rear connecting protrusion 326. In this arrangement the front undercut protrusion 322 of the RF tip retention mechanism 32 tessellates with the undercut recess 362 of the ceramic insulator 36, such that the RF tip retention mechanism cannot fall out or be pulled out easily. Further, the rear connecting protrusion 326, connects with the RF tip retainer 34, in a first manner, via the rear connecting recess 348 of the RF tip retainer 34. The RF tip retainer 34 consists of a front connection surface 342, front undercut protrusion 344, welding spots 346, rear connecting recess 348 and rear straight protrusion 350. The front connection surface 342 allows for connection of the RF tip retention mechanism 32 with the RF tip retainer 34 through welding at the welding spots 346. The front undercut protrusion 344 tessellates with the front undercut protrusion 322 and the undercut recess 362, to further ensure that the RF tip retention mechanism 32 and RF tip retainer 34 cannot fall out or be pulled out of the ceramic insulator 36 easily. The rear straight protrusion 350 tessellates with the straight recess 366 of the ceramic insulator 36. The rear straight protrusion 350 encompasses the electrical connection 38 for supplying RF power to the RF active tip.

Figure 4a shows a cross-sectional diagram of an RF hand instrument according to a further embodiment of the present disclosure. In the embodiment, the configuration is very similar to that displayed in Figure 3, however it provides a more robust mechanical tip interlock by utilising a double-undercut connection feature. Therefore, the configuration now ensures there is an undercut connection between the RF tip retention mechanism and the ceramic at the front of the instrument as well as an undercut connection between the RF tip retainer and the ceramic at the rear of the instrument.

Further, the RF hand instrument 40 or electrosurgical end effector 40 shown in Figure 4a, is composed of the RF tip retention mechanism 42, the RF tip retainer 44 and the ceramic insulator 46. The RF tip retention mechanism 42 consists of a front undercut protrusion 422 arrangement, a hole or passageway 424, a rear connecting recess 426 and a welding spot 428. In this arrangement the front undercut protrusion 422 of the RF tip retention mechanism 42 tessellates with the front undercut recess 462a of the ceramic insulator 46, such that the RF tip retention mechanism cannot fall out or be pulled out easily due to the front undercut recess surface 464 blocking movement. Further, the rear connecting recess 426, connects with the RF tip retainer 44, in a first manner, via the rear connecting protrusion 442 of the RF tip retainer 44. The RF tip retainer 44 consists of a rear connecting protrusion 442 and rear undercut protrusion 444. The rear undercut protrusion 444 tessellates with the rear undercut recess 462b of the ceramic insulator 46, such that the RF tip retention mechanism cannot fall out or be pulled out easily due to the rear undercut recess surface 468 blocking movement. The ceramic insulator 46 consists of the front undercut recess 462a, the front undercut recess surface 464, the rear undercut recess 462b, the rear undercut recess surface 466 and the hole or passageway 468. The passageway 469 of the ceramic insulator 46 aligns directly with the passageway 424 of the RF tip retention mechanism 42.

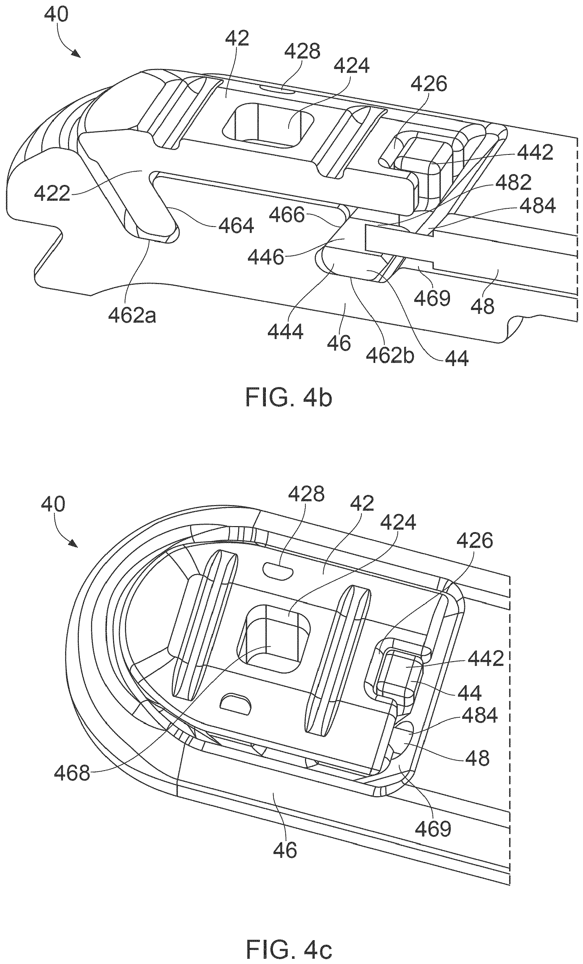

Figure 4b shows a further cross-sectional diagram of the RF hand instrument, which shows the electrical connection attached to the RF tip retainer. In this embodiment, the electrical connection is more exposed which would promote faster, more repeatable and more reliable erosion.

Further, the RF hand instrument 40 or electrosurgical end effector 40 shown in Figure 4b and 4a, is composed of the RF tip retention mechanism 42, the RF tip retainer 44, the ceramic insulator 46 and the electrical connection 48. Again, the RF tip retention mechanism 42 consists of a front undercut protrusion 422 arrangement, a hole or passageway 424, a rear connecting recess 426 and a welding spot 428. In this arrangement the front undercut protrusion 422 of the RF tip retention mechanism 42 tessellates with the front undercut recess 462a of the ceramic insulator 46, such that the RF tip retention mechanism cannot fall out or be pulled out easily due to the front undercut recess surface 464 blocking movement. Further, the rear connecting recess 426, connects with the RF tip retainer 44, in a first manner, via the rear connecting protrusion 442 of the RF tip retainer 44. The RF tip retainer 44 consists of a rear connecting protrusion 442, rear undercut protrusion 444 and electrical connection groove 446. Again, the rear undercut protrusion 444 tessellates with the rear undercut recess 462b of the ceramic insulator 46, such that the RF tip retention mechanism cannot fall out or be pulled out easily due to the rear undercut recess surface 466 blocking movement. The ceramic insulator 46 consists of the front undercut recess 462a, the front undercut recess surface 464, the rear undercut recess 462b, the rear undercut recess surface 466 and the electrical connection channel 469. The electrical connection 48 consists of the electrical connection tip 482 and the exposed electrical connection 484. The electrical connection 48 connects to the RF tip retainer 44 at the electrical connection tip 482. The electrical connection is exposed at the exposed electrical connection 484 position, in order to facilitate one method of increased rate of erosion of the electrical connection in comparison with the RF tip retention mechanism and RF tip retainer.

Figure 4c shows a top view of the cross-sectional diagram of the RF hand instrument which further shows the more exposed electrical connection via a deliberate enlarged window, in order to promote increased speed of erosion and improved repeatability/reliability. Also, the figure shows the laser welding locations for use in securing the RF tip retention mechanism and RF tip retainer together. This configuration provides the further advantages of increasing the mechanical retention and interlocking geometry, while deliberately exposing more of the active wire to plasma, and therefore erosion. This should help to ensure that the electrical connection always fails prior to the mechanical interlock, therefore always ensuring the instrument fails-safe.

Further, the RF hand instrument 40 or electrosurgical end effector 40 shown in Figure 4c, 4b and 4a, is composed of the RF tip retention mechanism 42, the RF tip retainer 44, the ceramic insulator 46 and the electrical connection 48. This top view of the RF hand instrument 40 depicts the connection between the rear connecting recess 426 of the RF tip retention mechanism and the rear connecting protrusion 442 of the RF tip retainer 44, more clearly. Also, the alignment of the RF tip retainer passageway 424 and the ceramic insulator passageway 468 is shown more clearly. Finally, the exposed electrical connection 484 can be seen due to the geometry of the electrical connection channel 469 in the ceramic insulator 46 to create the window to expose the electrical connection 48.

The devices disclosed herein can be designed to be disposed of after a single use, or they can be designed to be used multiple times. In either case, however, the device can be reconditioned for reuse after at least one use. Reconditioning can include a combination of the steps of disassembly of the device, followed by cleaning or replacement of particular pieces, and subsequent reassembly. In particular, the device can be disassembled, and any number of particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those of ordinary skill in the art will appreciate that the reconditioning of a device can utilize a variety of different techniques for disassembly, cleaning/replacement, and reassembly. Use of such techniques, and the resulting reconditioned device, are all within the scope of the present application.

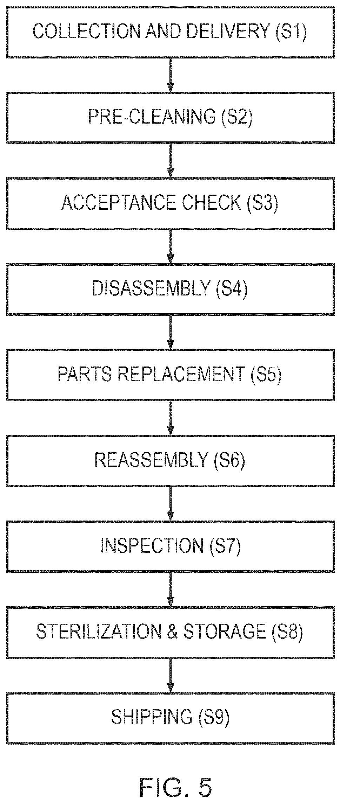

The treatment instrument 10 described above may be disposed of after one use, or may be repeatedly used a plurality of times. In the case of a configuration that is repeatedly used a plurality of times, for example, reprocessing method shown in FIG. 5 is required. An operator collects the used treatment instrument 10 after it has been used for treatment and transports it to a factory or the like (Step S1). Then, the operator cleans and sterilizes the collected and transported used treatment instrument 10 (Step S2). Next, the operator performs an acceptance check of the used treatment instrument 10 (Step S3). Subsequently, the operator disassembles the used treatment instrument 10 (Step S4) and replaces some parts of the used treatment instrument 10 with new parts (Step S5). After step S5, the operator assembles a new treatment instrument 10 (Step S6). In some examples, Step S6 can include adding an identifier to indicate the device has been modified from its original condition, such as a adding a label or other marking to designate the device as reprocessed, refurbished or remanufactured. After Step S6, the operator sequentially performs an inspection (Step S7), sterilization and storage (Step S8), and shipping (Step S9) of the new treatment instrument 10.

Preferably, the treatment instrument 10 described herein will be processed before surgery. First a new or used instrument is obtained and, if necessary, cleaned. The instrument can then be sterilized. In one sterilization technique, the instrument is placed in a closed and sealed container, such as a plastic or TYVEK^{®} bag. The container and instrument are then placed in a field of radiation that can penetrate the container, such as gamma radiation, x-rays, or higher energy electrons. The radiation kills bacteria on the instrument and in the container. The sterilized instrument can then be stored in the sterile container. The sealed container keeps the instrument sterile until it is opened in the medical facility. The device may also be sterilized using any other technique known in the art, including but limited to beta or gamma radiation, ethylene oxide, or steam.

Various modifications, whether by addition, substitution, or deletion will be apparent to the intended reader to provide further embodiments of the present disclosure, any and all of which are intended to be encompassed by the appended claims.

## Claims

1. An electrosurgical end effector, comprising a tissue treatment electrode mounted in an insulative instrument tip, the end effector further comprising an electrical connection to supply RF energy to a tip of the tissue treatment electrode, the arrangement being such that the electrical connection is arranged to fail in use prior to any failure of the tissue treatment electrode.

2. The electrosurgical end effector of claim 1, wherein the electrical connection erodes in use faster than the tissue treatment electrode.

3. The electrosurgical end effector of any preceding claims, wherein the tissue treatment electrode is separate to the electrical connection.

4. The electrosurgical end effector of any preceding claims, wherein the electrical connection is made from an electrically conductive material which erodes faster than the RF tip in the same plasma conditions.

5. The electrosurgical end effector of any preceding claims, wherein the electrical connection has a greater area exposed to RF plasma by virtue of the insulative instrument tip geometry.

6. The electrosurgical end effector of any preceding claims, wherein an enlarged open window over the electrical connection is present in the insulative instrument tip to promote increased erosion levels in this area compared to the tissue treatment electrode.

7. The electrosurgical end effector of any preceding claims, wherein the electrical connection has a thin geometry.

8. The electrosurgical end effector of any preceding claims, wherein the end effector further comprises a saline suction passageway which draws hot saline over the electrical connection to increase erosion levels in this area compared to the tissue treatment electrode.

9. The electrosurgical end effector of any preceding claims, wherein the tip of the tissue treatment electrode is still mechanically strong after the electrical connection has fully eroded, such that the tissue treatment electrode can be removed from the patient without the tip of the tissue treatment electrode becoming detached.

10. The electrosurgical end effector of any preceding claims, wherein the components of the tissue treatment electrode are welded together in a configuration that prevents the removal of the tissue treatment electrode from the insulative instrument tip.

11. The electrosurgical end effector of any preceding claims, wherein a user is alerted when the electrical connection has been fully eroded, and RF function has been lost.

12. The electrosurgical end effector of any preceding claims, wherein the electrical connection erodes to failure in use beyond a time considered normal use.

13. An RF hand instrument tip configuration, comprising a RF tip and a retention means mounted in a ceramic insulator, wherein, the RF tip and the retention means further comprise an electrical connection to supply RF energy to the RF tip, the arrangement being such that the electrical connection reliably erodes at a faster rate than the RF tip or retention means.

14. The RF hand instrument tip configuration of claim 13, wherein the retention means comprises an RF tip retainer and an RF tip retention mechanism.

15. The RF hand instrument tip configuration of claim 13 or 14, wherein the electrical connection is made to reliably erode faster than the RF tip and retention means by:
making the electrical connection from an electrically conductive material which erodes faster than the RF tip in the same plasma conditions; and/or
designing the ceramic insulator geometry such that an enlarged open window over the electrical connection is present to promote increased erosion levels in this area; and/or
designing the electrical connection to have a thin or narrow geometry; and/or
designing a saline suction passageway which draws hot saline over the electrical connection to increase erosion levels in this area.
